# EUROPEAN PATENT SPECIFICATION

(11) **EP 2 713 965 B1**
(45) Date of publication and mention of the grant of the patent: **18.11.2015**
(21) Application number: 12727489.2
(22) Date of filing: 30.05.2012
(51) Int. Cl.: A61F 6/18, A61F 6/22, A61B 17/42

(54) **SYSTEMS FOR REDUCING FLUID LEAKAGE AND SPRAY-BACK FROM ENDOSCOPIC MEDICAL PROCEDURES**
SYSTEME ZUR VERRINGERUNG VON FLÜSSIGKEITSAUSLAUF UND RÜCKSPRÜHEN BEI ENDOSKOPISCHEN MEDIZINISCHEN VERFAHREN
SYSTÈME DE REDUCTION DES FUITES DE FLUIDE ET DE JETS ARRIÈRE LORS D'ACTES MEDICAUX ENDOSCOPIQUES

(30) Priority: 31.05.2011 US 201113149631; 12.12.2011 US 201113323741
(43) Date of publication of application: 09.04.2014
(73) Proprietor: Bayer Healthcare LLC, Whippany, NJ 07981-0915 (US)
(72) Inventor: STOUT, Christopher, A., San Bruno, CA 94066 (US); SWANN, Betsy, Grass Valley, CA 95945 (US); CRUZADA, Julian, San Jose, CA 95126 (US); SEPE, Chris, Campbell, CA 95008 (US); SLOAN, Robert, Todd, Saratoga, CA 95070 (US)
(74) Representative: BIP Patents
(86) International application number: PCT/US2012/040003
(87) International publication number: WO 2012/166799

(56) References cited:
- US-A- 5 749 889
- US-A1- 2003 009 128
- US-A1- 2005 004 512
- US-A1- 2005 085 880
- US-A1- 2006 293 560
- US-A1- 2008 154 256

## Description

### BACKGROUND

Embodiments of the present invention relate to the field minimally invasive surgical medical devices. More specifically, embodiments of the present invention relate to devices for transcervical gynecological procedures.

Female contraception and sterilization may be effected by transervically introducing an object into a fallopian tube to inhibit conception. Devices, systems and methods for such a contraceptive approach have been described in various patents and patent applications assigned to the present assignee. For example, U.S. Patent No. 6,526,979, U.S. Patent No. 6,634,361, U.S. Patent No. 2006/0293560, which is regarded as the closest prior art, and U.S. Patent No. 2011/094519 describe transcervically inserting an insert (also referred to as implant and device) into an ostium of a fallopian tube and mechanically anchoring the insert within the fallopian tube. One example of such an assembly is known as "Essure" ® from Conceptus, Inc. of Mountain View, California. Tissue in-growth into the "Essure" ® insert provides long-term contraception and/or permanent sterilization without the need for surgical procedures.

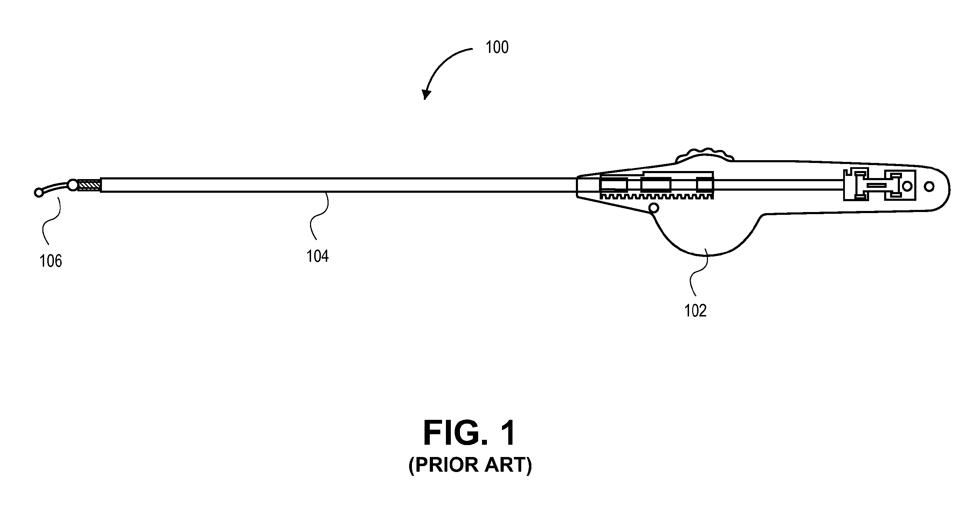

The insert may be delivered to the fallopian tube with a delivery catheter assembly such as the one illustrated in FIG. 1. The delivery catheter assembly 100 is formed of a control device 102 such as a handle, an elongated sheath 104, and an insert 106. The delivery catheter assembly 100 may be transcervically positioned into the uterus and the fallopian tubes via a hysteroscope system. Advancement of the delivery catheter system within the uterus and the fallopian tubes is usually facilitated by distending the uterus with a distention fluid, such as saline, and viewing the placement with the hysteroscope system.

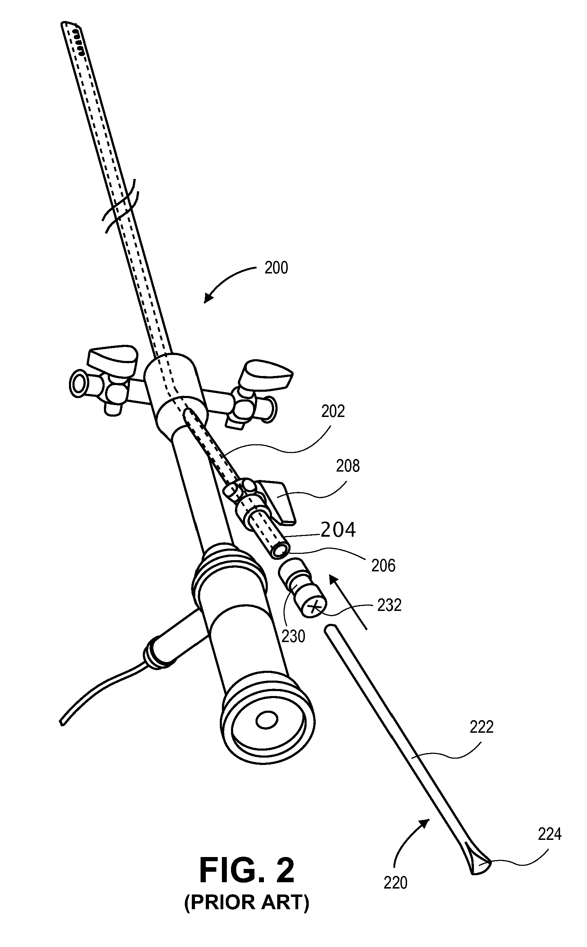

Referring to FIG. 2 the hysteroscope system 200 may include a nozzle 204 including a valve clamp 208, such as a ball valve clamp, and an access port 206 positioned at a proximal end of the nozzle 204 to access a working channel 202 into which the delivery catheter assembly is inserted.. Closing the valve clamp 208 may seal the entrance of the working channel 202 to prevent a distention fluid from leaking out of the access port 206 when a delivery catheter assembly does not occupy the working channel of the hysteroscope system. A sealing cap 230 including a proximal seal 232 can be fitted over the nozzle 204 containing the access port 206 to prevent distention fluid from leaking out of the hysteroscope system when a delivery catheter assembly occupies the working channel of the hysteroscope system.

An introducer 220 may be used in order to prevent damaging the tip of the elongated sheath 104 or insert 106 of the delivery catheter assembly 100 during insertion through the proximal seal 232 of the sealing cap 230 and access port 206, and into the working channel 202 of the hysteroscope system 200. Introducer 220 includes a sheath portion 222 and slit opening 224 to aid in grasping and in the removal of the introducer 220. The introducer 220 is inserted through the proximal seal 232 of the sealing cap 230 and into the working channel 202 prior to inserting the delivery catheter assembly 100. When the introducer 220 is inserted through the sealing cap 230, fluid can spray out of the introducer 220 and onto the physician or physician's assistant. The amount of fluid spray-back can be significant depending on the distention fluid pressure during the procedure.

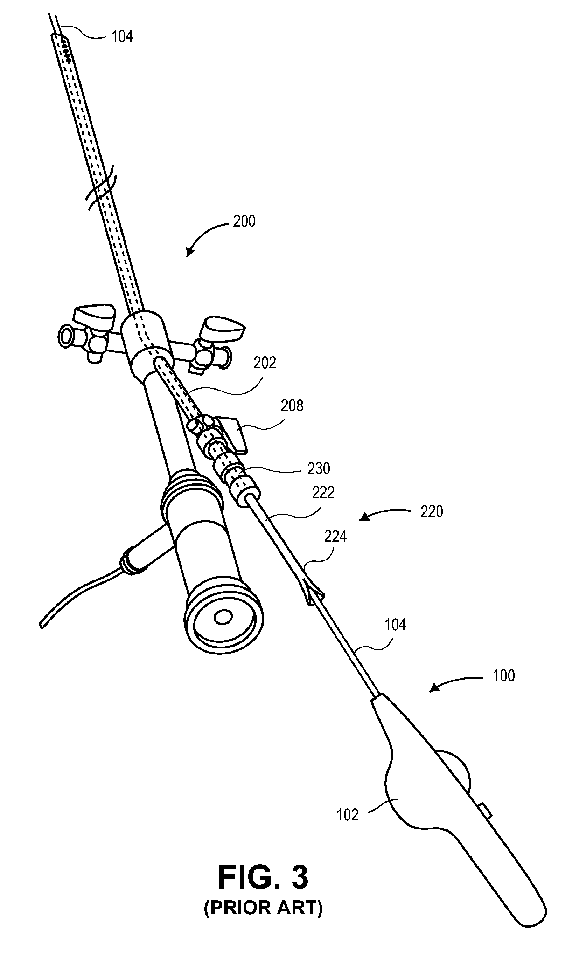

Referring to FIG. 3, after placing the introducer 220 into the working channel 202, the tip of delivery catheter assembly 100 is inserted into the slit opening 224 and through the sheath 222 of the introducer 220 in order to advance the delivery catheter assembly 100 into the working channel 202 of the hysteroscope system. This is typically performed as soon as possible after placement of the introducer 220 into the working channel 202 in order to minimize the amount of fluid spray-back from the introducer. The introducer 220 may then be removed or may be kept in place throughout the procedure. After insertion of the delivery catheter assembly 100 into the introducer 200, an amount of distention fluid may still leak from between the introducer and elongated sheath 104 of the delivery catheter assembly 100, as well as from the between the sealing cap 230 and nozzle 204.

### SUMMARY

Embodiments of the present invention generally provide assemblies for inserting a delivery catheter assembly into a working channel of an endoscope, such as a hysteroscope system for accessing a female reproductive system. While embodiments of the invention are described with reference to a hysteroscope system, it is understood that the embodiments are not limited to such and may also be compatible with other optical surgical devices and endoscope systems. In one aspect, embodiments of the invention describe a tip protector sleeve which functions as an introducer and protects the tip of a delivery catheter assembly when piercing a sealing cap, as well as during insertion through an access port, into the working channel and past a valve clamp of a hysteroscope system. In another aspect, embodiments of the invention describe a system which may reduce the amount of fluid spray-back and leakage associated with inserting a delivery catheter assembly into the working channel of a hysteroscope system. In yet another aspect, embodiments of the invention describe a sealing cap which includes a double seal that may prevent leakage and spray-back when the sealing cap is fitted over a nozzle containing the access port to the working channel of a hysteroscope system both when a delivery catheter assembly is, and is not inserted into the sealing cap. The sealing cap may additionally be compatible with a plurality of commercially available hysteroscope systems having nozzles of different outside dimensions.

One embodiment of the present invention relates to a delivery catheter assembly which may be used to deliver an insert to an ovarian pathway (e.g. a fallopian tube) of a female body. The delivery catheter assembly may include a control device, an elongated catheter sheath having a distal end and a proximal end connected to the control device, and a tip protector sleeve. The tip protector sleeve may be locked onto the elongated catheter sheath and slideable over a length of the elongated catheter sheath between a proximal-stop position and a distal-stop position along the elongated catheter sheath. The delivery catheter assembly may further include an interference stop which determines the distal-stop position and prevents the tip protector sleeve from sliding off of the distal end of the elongated catheter sheath. For example, the interference stop may include a male interference part which interferes with sliding of a female interference part over the elongated catheter sheath. The male interference part may be fixed to the elongated catheter sheath, and the tip protector sleeve may comprise the female interference part. The tip protector sleeve may additionally incorporate a sealing valve to reduce the amount of fluid spray-back and leakage associated with inserting the delivery catheter assembly into the working channel of a hysteroscope system

It is herein described a method of forming a delivery catheter assembly which includes sliding a tip protector sleeve over a distal end of an elongated catheter sheath and toward a control device, and then fixing a bump onto a distal region of the elongated catheter sheath. Alternatively, the bump may be fixed onto the distal region of the elongated catheter sheath, and then the tip protector sleeve is slid over a proximal end of the elongated catheter sheath toward the bump prior to attaching the control device to the elongated catheter sheath. The control device may prevent the tip protector sleeve from sliding off a proximal end of the elongated sheath and define, in part, a proximal-stop position. The bump may prevent the tip protector sleeve from sliding off of a distal end of the elongated catheter sheath and define, in part, a distal-stop position. In an embodiment, the bump may be fixed onto a distal region of the elongated catheter sheath by crimping a band onto the elongated catheter sheath. It is not necessary to crimp the entire length of the band, and only a proximal end of the band is crimped onto the elongated catheter shaft in an embodiment.

It is herein described a method of delivering an insert into a body lumen such as an ovarian pathway (e.g. a fallopian tube) of a female body. Utilizing a delivery catheter assembly in accordance with embodiments of the invention the tip protector sleeve is positioned at the distal-stop position, and the tip protector sleeve is inserted through a proximal seal of a sealing cap, through an access port of a hysteroscope system and into a working channel of the hysteroscope system. The distal end of the elongated catheter sheath and insert are inserted through the proximal seal of the sealing cap, through the access port and into the working channel of the hysteroscope system simultaneously with the tip protector sleeve in the distal-stop position. The distal end of the elongated catheter sheath and insert may then be advanced through the tip protector sleeve and beyond the hysteroscope system to a target location within the body lumen where the insert is deployed within the body lumen. The tip protector sleeve is advanced through the sealing cap into the working channel until a flanged mechanical stop, such as a bead or flared portion, abuts the sealing cap (or access port if a sealing cap is not utilized) prior to advancing the elongated sheath and insert to the target location.

The present invention relates to a sealing cap which is configured to reduce fluid leakage and spray-back. The sealing cap may include a double seal formed of a proximal seal and a self closing seal, and a distal seal which is configured to be fitted over a nozzle housing a working channel of a hysteroscope system. In the double seal, the proximal seal may be configured to reduce fluid leakage when a delivery system is inserted through the proximal seal, and the self closing seal may be configured to reduce fluid leakage when a delivery system is not inserted into the sealing cap. The sealing cap may additionally be configured to be compatible with a plurality of commercially available hysteroscope systems having nozzles of different outside dimensions or diameters. In such an embodiment, the sealing cap may including a plurality of shoulders of different dimensions (e.g. widths or diameters) and a separation chamber distally adjacent to the self closing seal which spatially separates the self closing seal from the plurality of shoulders.

Another embodiment of the present invention relates to a kit which may include a delivery catheter assembly and a sealing cap. In such an embodiment, the delivery catheter assembly may include a control device, an elongated catheter sheath having a distal end and a proximal end connected to the control device, and a tip protector sleeve locked onto the elongated catheter sheath as previously described. The sealing cap may have a proximal seal, a self closing seal, and a distal seal as previously described. The sealing cap may additionally be configured to be compatible with a plurality of commercially available hysteroscope systems having nozzles of different outside dimensions as previously described.

Another embodiment of the present invention relates to a kit which may include a delivery catheter assembly as previously described, an introducer, and a sealing cap. In such an embodiment, the introducer may include an elongated shaft and a valve which is configured to be self closing. In one embodiment, the sealing cap includes a proximal seal to receive and form a seal around the introducer and a distal seal to be fitted over and form a seal around a nozzle housing a working channel of a hysteroscope system. In another embodiment, the sealing cap may have a proximal seal, a self closing seal, and a distal seal as previously described. The sealing cap may additionally be configured to be compatible with a plurality of commercially available hysteroscope systems having nozzles of different outside dimensions as previously described.

### BRIEF DESCRIPTION OF THE DRAWINGS

FIG. 1 is a cross-sectional side view illustration of a delivery catheter assembly.
FIG. 2 is an isometric view illustration of a hysteroscope system and an introducer.
FIG. 3 is an isometric view illustration of a delivery catheter assembly inserted into an introducer and working channel of a hysteroscope system.
FIG. 4 is a side view illustration of a tip protector sleeve in accordance with an embodiment of the invention.
FIG. 5A is a side view illustration of a delivery catheter assembly with a tip protector sleeve at a proximal-stop position in accordance with an embodiment of the invention.
FIG. 5B is a side view illustration of a delivery catheter assembly with a flanged mechanical stop of a tip protector sleeve in a cavity of a control device in accordance with an embodiment of the invention.
FIG. 5C is a side view illustration of a delivery catheter assembly with a tip protector sleeve fastened into the proximal-stop position by a friction fitting in accordance with an embodiment of the invention.
FIG. 5D is a side view illustration of a delivery catheter assembly with a tip protector sleeve screwed into the proximal-stop position in accordance with an embodiment of the invention.
FIG. 6 is a side view illustration of a delivery catheter assembly with a tip protector sleeve at a distal-stop position in accordance with an embodiment of the invention.
FIG. 7 is a close-up side view illustration of the proximal end of a tip protector sleeve over an elongated catheter sheath in accordance with an embodiment of the invention
FIG. 8 is a close-up cross-sectional side view illustration of the proximal end of a tip protector sleeve over an elongated catheter sheath in accordance with an embodiment of the invention
FIG. 9 is a cross-sectional side view illustration of a tip protector sleeve in accordance with an embodiment of the invention.
FIG.10 is a cross-sectional side view illustration of a tip protector sleeve in accordance with an embodiment of the invention.
FIGS. 11A-11C are cross-sectional side view illustrations of tip protector sleeves incorporating various sealing valves in accordance with embodiments of the invention.
FIGS. 12A-12C are isometric view illustrations of inserting a delivery catheter assembly into a working channel of a hysteroscope system in accordance with an embodiment of the invention.
FIG. 13A is a cross-sectional side view illustration of a sealing cap in accordance with an embodiment of the invention.
FIG. 13B is an isometric view illustration of a sealing cap in accordance with an embodiment of the invention.
FIGS. 14A-14B are top view illustrations of self closing seals in accordance with embodiments of the invention.
FIG. 14C is a side view illustration of a self closing seal in accordance with an embodiment of the invention.
FIGS. 15A-15B are close-up side view illustrations of a proximal seal in accordance with embodiments of the invention.
FIG. 16 is a cross-sectional side view illustration of a sealing cap in accordance with embodiments of the invention.
FIGS. 17A-17C are side view illustrations of various nozzles with different outside dimensions in accordance with embodiments of the invention.
FIGS. 17D-17F are cross-sectional side view illustrations of a sealing cap fitted over various nozzles with different outside dimensions in accordance with embodiments of the invention.
FIGS. 18-19 are side view illustrations of kits in accordance with embodiments of the invention.

### DETAILED DESCRIPTION

Embodiments of the present invention generally provide assemblies for inserting a delivery catheter assembly into a working channel of an endoscope, such as a hysteroscope system or other optical surgical device for accessing a female reproductive system. Various embodiments and aspects will be described with reference to details discussed below and the accompanying drawings will illustrate the various embodiments. The following description and drawings are illustrative of the invention and are not to be construed as limiting the invention. Numerous specific details are described to provide a thorough understanding of various embodiments of the present invention. However, in certain instances, well-known or conventional details are not described in order to provide a concise discussion of embodiments of the present invention.

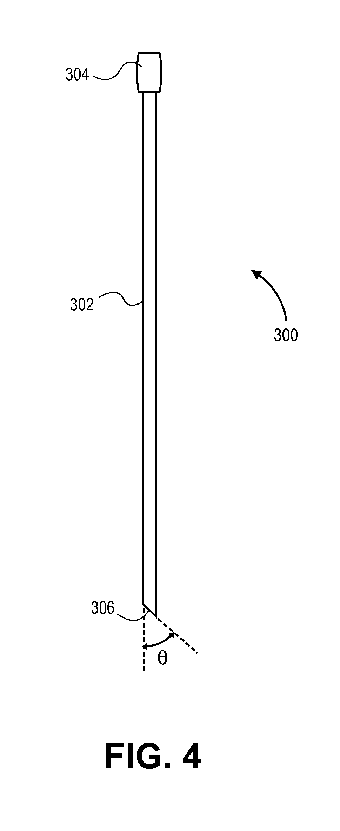

In an embodiment, a delivery catheter assembly includes a control device, an elongated catheter sheath having a distal end and a proximal end connected to the control device, and a tip protector sleeve. Referring to the FIG. 4 the tip protector sleeve 300 may include an elongated shaft 302, a flanged mechanical stop 304 at a proximal end, and a distal end 306. The distal end 306 can be flat or angled to assist with piercing of a sealing cap. In an embodiment, the distal end 306 has an approximately 45 degree angled tip. Elongated shaft 302 may be formed of a material and to a thickness which can be molded and does not buckle when piercing a sealing cap. For example, elongated shaft 302 may be formed of a material such as polyether ether ketone (PEEK).

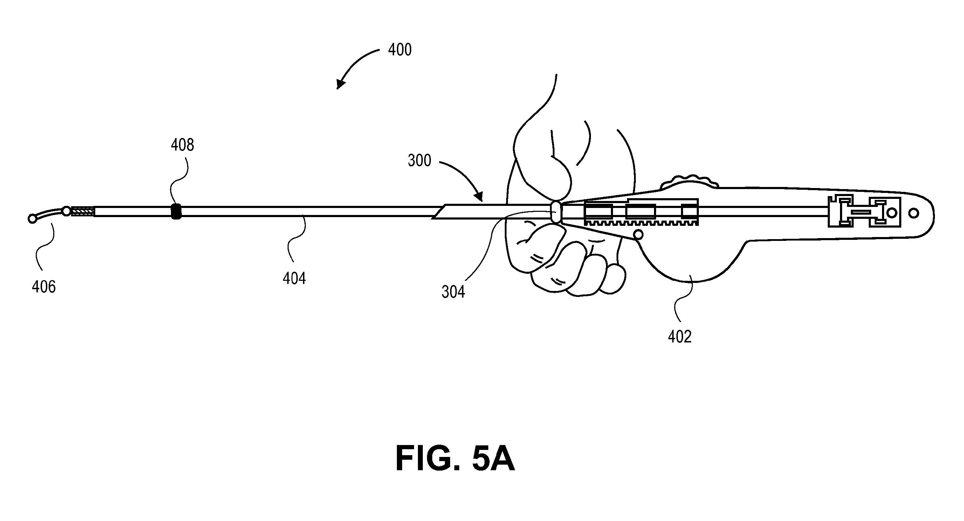

Flanged mechanical stop 304 may provide variety of functions, be formed of a variety of materials and have a variety of shapes and sizes as will be explained in further detail with regard to FIGS. 5A-12C. For example, flanged mechanical stop 304 may be formed of a moldable material such as polycarbonate, or from the same material as the elongated shaft 302. Flanged mechanical stop 304 may be sized and shaped larger than the inside diameter (ID) of a corresponding access port opening to a working channel or proximal seal of a sealing cap if present in order to act as a stop mechanism that controls the insertion depth of the tip protector sleeve 300 into the working channel. Flanged mechanical stop 304 may also be sized and shaped to be gripped by an operator's hand to assist with sliding of the tip protector sleeve 300 over a length of the elongated catheter sheath 404 of a catheter assembly. In this respect, one function may be as a handle at the proximal end of the tip protector sleeve 300. Flanged mechanical stop 304 may also incorporate a sealing valve to reduce the amount of fluid spray-back and leakage associated with inserting the delivery catheter assembly into a working channel.

Referring to FIGS. 5A-6, a delivery catheter assembly 400 in accordance with embodiments of the invention is illustrated in which the tip protector sleeve 300 is locked onto and slideable over a length of the elongated catheter sheath 404. The delivery catheter assembly 400 may be formed by sliding a tip protector sleeve 300 over a distal end of an elongated catheter sheath 404 and toward a control device 402, and then fixing a bump 408 onto a distal region of the elongated catheter sheath 402. Alternatively, the bump 408 may be fixed onto the distal region of the elongated catheter sheath 402, and then the tip protector sleeve 300 is slid over a proximal end of the elongated catheter sheath 402 toward the bump 408 prior to attaching the control device 402 to the elongated catheter sheath 402. The control device 402 may prevent the tip protector sleeve 300 from sliding off a proximal end of the elongated sheath 402 and define, in part, a proximal-stop position. The bump 408 may prevent the tip protector sleeve 300 from sliding off of a distal end of the elongated catheter sheath 402 and define, in part, a distal-stop position. An operator may grip the flanged mechanical stop 304 by hand, for example between a thumb and index finger, and slide the tip protector sleeve over the elongated catheter sheath 404 between the proximal-stop and distal-stop positions.

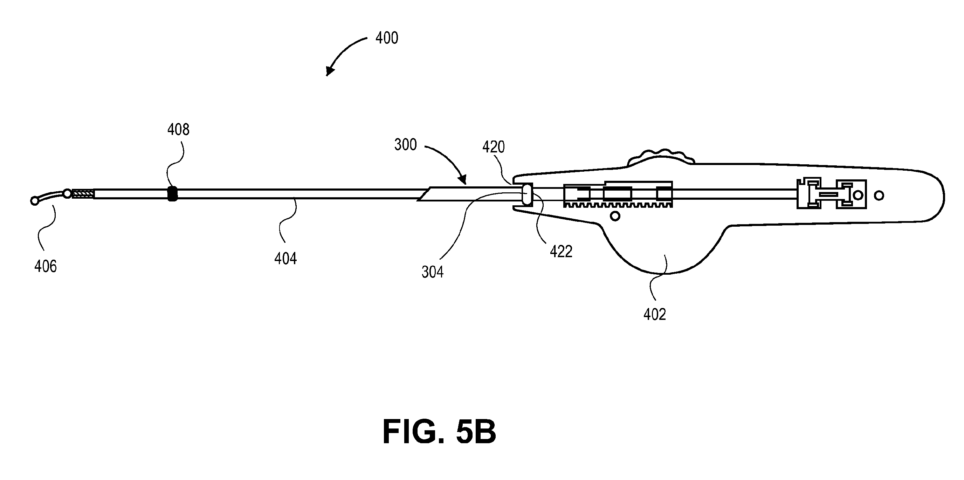

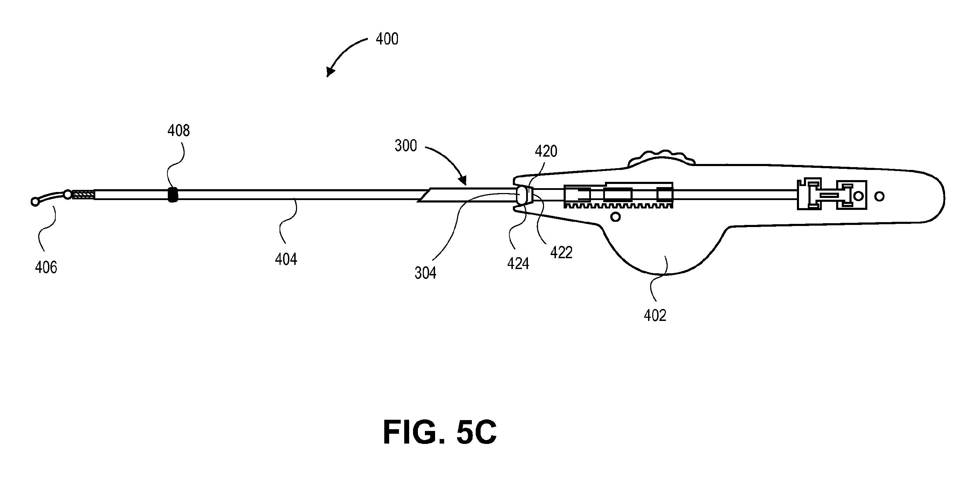

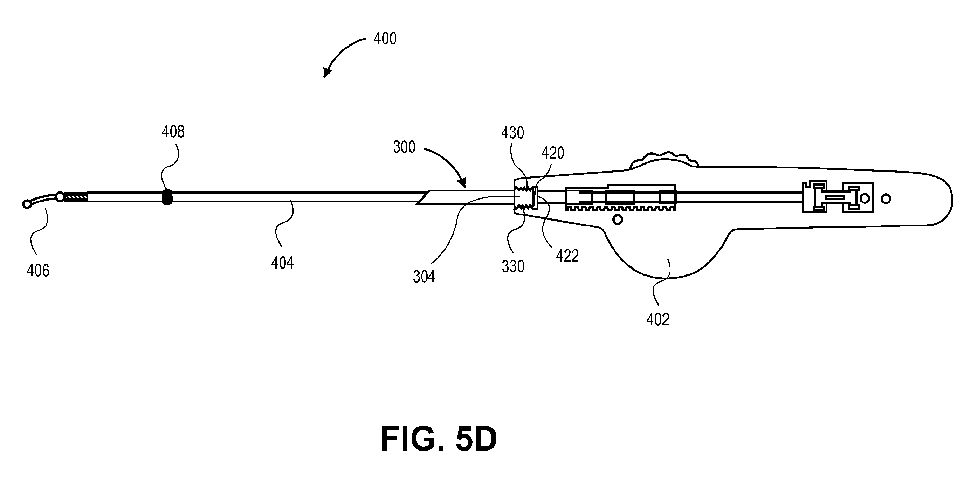

FIG. 5A is an illustration of the tip protector sleeve 300 positioned at a proximal-stop position. In the embodiment illustrated in FIG. 5A, the flanged mechanical stop 304 abuts a distal end of the control device 402, though other proximal-stop positions along the elongated catheter sheath 404 are contemplated in accordance with embodiments of the invention. For example, FIGS. 5B-5D are illustrations of embodiments in which the control device 402 is configured to allow flanged mechanical stop 304 to slide within a cavity 420 located at a distal portion of the control device 402. Such embodiments may be useful during operation in order to utilize the full working length of the elongated catheter sheath 404. In this manner, the distal end of the control device 402 can be advanced to abut the access port 206 of the hysteroscope system or the proximal end of a sealing cap 230 if desired during operation. Alternatively, the distal end of the control device 402 can be advanced over the access port 206 of the hysteroscope system or over sealing cap 230 and the flanged mechanical stop 304 is allowed to abut the access port 206 or the proximal end of the sealing cap 230. As illustrated in FIG. 5B, flanged mechanical stop 304 may be slid into cavity 420 to abut a back wall 422 of the cavity at the proximal-stop position. Flanged mechanical stop 304 may be also configured to fasten onto the handle 402 at the proximal-stop position. For example, FIG. 5C is an illustration of an embodiment in which flanged mechanical stop 304 may be slid into cavity 420 and fastened into the proximal-stop position by a friction fitting with sloped walls 424 of the cavity. FIG. 5D is an illustration of an embodiment in which flanged mechanical stop 304 may be screwed into cavity 420 in which threads 330 on flanged mechanical stop 304 mate with threads 430 inside cavity 420 to fasten tip protection sleeve 300 in the proximal-stop position. In an embodiment, a suitable fastening mechanism for fastening tip protector sleeve 300 onto control device 402 is able to hold the tip protector sleeve 300 in the proximal-stop position during withdrawal of the delivery catheter assembly 400 from the working channel of the hysteroscope system.

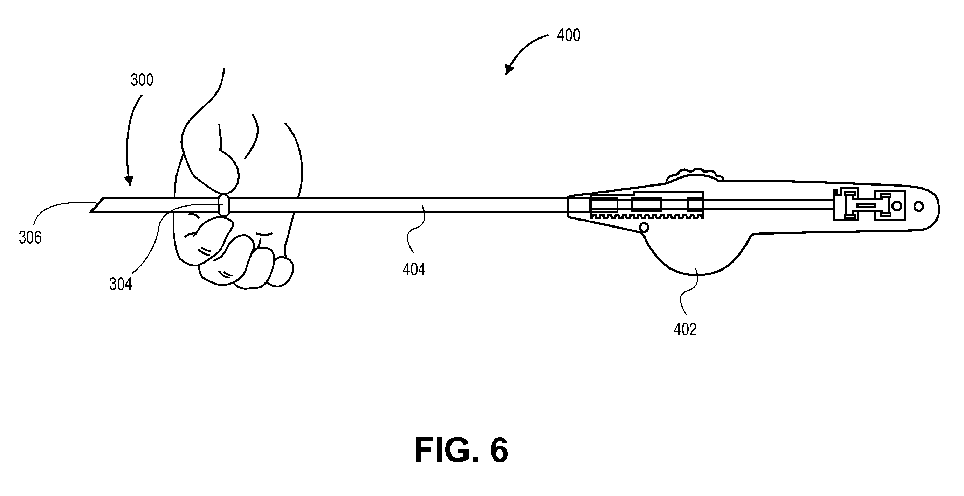

Referring now to FIG. 6 an operator may slide the tip protector sleeve over the elongated catheter sheath 404 between the proximal-stop position and the distal-stop position illustrated in FIG. 6. As illustrated, the distal end 306 of tip protector sleeve 300 may extend distally beyond a distal end of the elongated catheter sheath 404 and insert 406 when at the distal-stop position. In this manner, the tip protector sleeve 300 may protect the distal ends of the elongated catheter sheath 404 and insert 406 during piercing of a sealing cap and during insertion into the working channel and past a valve clamp of the hysteroscope system.

An interference stop may determine the distal-stop position and prevent the tip protector sleeve 300 from sliding off of the distal end of the elongated catheter sheath 404. In an embodiment, the interference stop includes a male interference part which interferes with sliding of a female interference part over the elongated catheter sheath. Referring again to FIG. 5A, the male interference part may comprise a bump 408 fixed to the elongated catheter sheath 404. Bump 408 may be formed along only a portion of the circumference of the elongated sheath, or may encircle the circumference of the elongated sheath. In an embodiment, bump 408 is a band fixed to and encircling the circumference of the elongated catheter sheath. In an embodiment, bump 408 is fixed to the elongated catheter sheath 404 with a sufficient shear strength to ensure that the tip protector sleeve may be removed from a working channel of a hysteroscope system along with removal of the elongated catheter sheath 404.

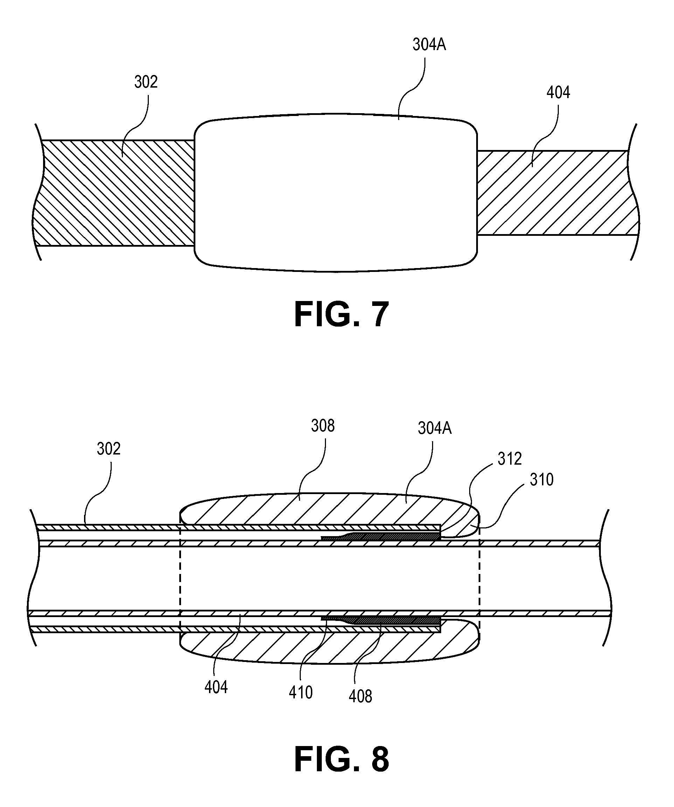

FIG. 7 is a close-up side view illustration of the proximal end of a tip protector sleeve over an elongated catheter sheath in accordance with an embodiment of the invention. FIG. 8 is a close-up cross-sectional side view illustration of the proximal end of a tip protector sleeve over an elongated catheter sheath in accordance with an embodiment of the invention. As illustrated in FIGS. 7-8, flanged mechanical stop may be a bead 304A having a barrel-like shape, though embodiments of the invention are not limited to such a shape. In an embodiment, bead 304A is fixed to shaft 302 with an adhesive.

Referring to FIG. 8, in an embodiment bead 304A may be fixed to a proximal end of the elongated shaft 302. Bead 304A may include a distal portion 308 surrounding the proximal end of the elongated shaft 302, a shoulder portion 310 extending proximally of the elongated shaft 302, and a backstop 312. The distal portion 308 may be fixed to the elongated shaft 302 with an adhesive. In an embodiment, the backstop 312 abuts the proximal end of the elongated shaft 302. Backstop 312 may also have a height which is greater than a thickness of the elongated shaft 302. For example, the height may be defined as the distance between and inside diameter (ID) of the backstop and an ID of the distal portion 308 of the bead 304A. In accordance with various embodiments of the invention, the dimensions and location of the backstop 312 as they relate to the dimensions and location of bump 410 create an interference stop which determines the distal-stop position and prevents the tip protector sleeve from sliding off of the distal end of the elongated catheter sheath 404.

In an embodiment, the tip protector sleeve 300 is locked onto an "Essure" ® delivery catheter assembly. In such an embodiment, the ID of elongated catheter sheath 404 may be between 0.1029 and 0.1067 cm (0.0405 and 0.0420 inches) and the outside diameter (OD) of elongated catheter sheath 404 may be between 0.1367 and 0.1422 cm (0.0538 and 0.0560 inches). Elongated catheter sheath 404 may be formed of a polyether block amide also known under the trade name PEBAX. The OD of elongated catheter sheath 404 may be used to determine the ID of bump 408. In an embodiment, bump 408 may have an ID between 0.1384 and 0.1410 cm (0.0545 and 0.0555 inches) and an OD between 0.1461 and 0.1473 cm (0.0575 and 0.0580 inches). In one embodiment, the ID of bump 408 may be smaller than the OD of the elongated catheter sheath 404. In another embodiment, the OD of the elongated catheter sheath 404 is smaller than the ID of bump 408. For example, the OD of the elongated catheter sheath 404 may be between 0.1367 and 0.1377 cm (0.0538 and 0.0542 inches).

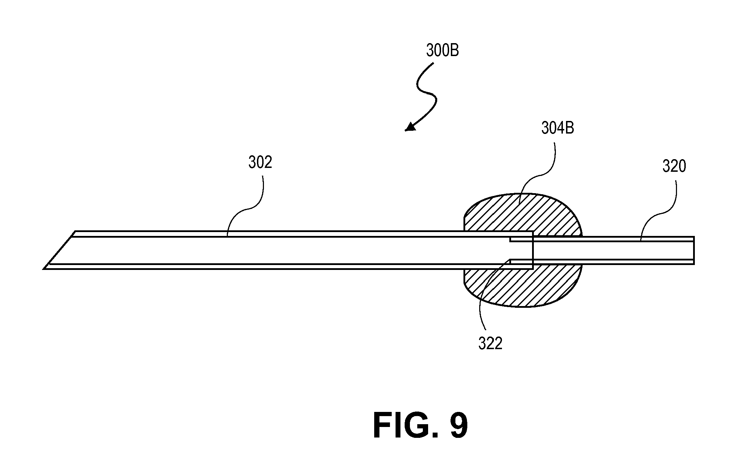

Bump 408 may be a band that is fixed to and encircles the elongated catheter sheath. Bump 408 may be fixed to the elongated catheter sheath 404 by a variety of mechanisms including adhesive and crimping. In an embodiment, bump 408 is formed of a material which is strong enough to resist deformation during operation of the delivery catheter assembly, yet malleable enough to be suitable for crimping. For example, stainless steel possesses suitable strength and malleability. In an embodiment, only a distal end 410 of the band is crimped onto the elongated catheter sheath, as illustrated in FIG. 8. This leaves the proximal end, with the original OD between 0.1461 and 0.1473 cm (0.0575 and 0.0580 inches) to act as the male interference part which interferes with the ID of backstop 312 functioning as a part of the female interference part. While an embodiment of bump 408 is described in detail in FIG. 8 in operable relationship with bead 304A, it is understood that bump 408 can be in operable relationship with other flanged mechanical stops, such as those illustrated in FIGS. 9-11C.

Still referring to FIG. 8, bead 304A may be formed of a variety of materials and have a variety of shapes and sizes to perform a variety of functions. In one aspect, bead 304A may be sized and shaped larger than the inside diameter (ID) of a corresponding access port opening to a working channel or proximal seal at the proximal end of a sealing cap if present in order to act as a stop mechanism that controls the insertion depth of the tip protector sleeve 300 into the working channel. In one aspect, bead 304A may perform the function as a handle for gripping by the operator. In another aspect, bead 304A may include a backstop 312 which functions as part of the female interference part. In an embodiment, bead 304A has an OD of approximately 0.284 cm (0.112 inches). The distal portion 308 of bead 304A may have an ID of approximately 0.178 cm (0.070 inches) and may be bonded to the OD of elongated shaft 302. Backstop 312 may have an ID which is smaller than an OD of the proximal end of bump 408. For example, backstop 312 may have an ID between 0.1435 and 0.1461 (0.0565 and 0.0575 inches). In such an embodiment, backstop 312 has a height that extends from the ID of back stop 312 to the ID of the distal portion 308 of the bead 304, or approximately 0.033 cm (0.013 inches).

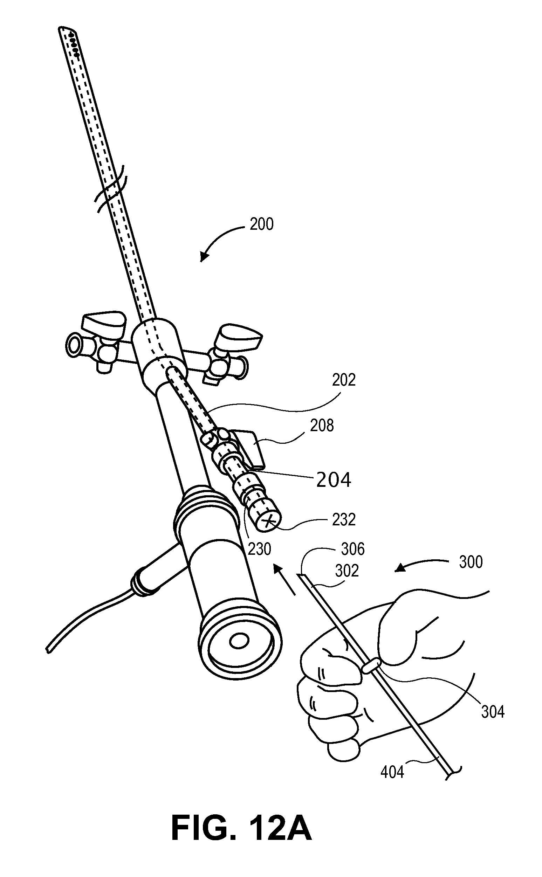

In accordance with embodiments of the invention tip protector sleeve 300 may be locked onto the delivery catheter assembly 400. Shaft 302 may be used to pierce a sealing cap and protect the tip of the insert 406, elongated catheter sheath 404 or guidewire during insertion into the working channel and past a valve clamp of a hysteroscope system. In an embodiment, the shaft 302 and elongated catheter sheath 404 may be advanced into a working channel of a hysteroscope system without allowing a significant amount of fluid (e.g. saline) spray-back or leakage from the delivery catheter assembly. The elongated catheter sheath 404 may additionally be slid through the shaft 302 to deliver the insert 406 to a body lumen, while the delivery catheter assembly 400 does not allow a significant amount of fluid leakage. Referring now to FIG. 12A, the shaft 302 may pierce the proximal seal 232 at the proximal end of a sealing cap 230 with the tip of the shaft 306. The outside diameter of the shaft 302 may fit tightly in the proximal seal 232 effectively creating a water tight seal between the sealing cap 230 and tip protector sleeve 300.

The reduction of fluid spray-back and leakage may also be achieved by controlling the shape and dimensions of the tip protector sleeve 300 as it interacts with the elongated catheter sheath 404 and bump 408. In an embodiment, shaft 302 may be approximately 7.16 cm (2.82 inches) long from the proximal end to the distal end of the tip 306, which may be angled. Shaft 302 may have an ID between 0.1486 and 0.1537 cm (0.0585 and 0.0605 inches) and an OD between 0.1753 and 0.1803 cm (0.0690 and 0.0710 inches). The shaft 302 ID maybe selected to not allow for fluid to flow proximally between the shaft 302 and elongated catheter sheath 404 (and bump 408), while still allowing for the elongated catheter sheath 404 to slide and be advanced through the shaft 302. In such and embodiment, a minimum clearance between the ID of the elongated shaft 302 (e.g. 0.150 cm (0.059 inches)) and the OD of the elongated catheter sheath 404 (e.g. 0.140 cm (0.055 inches)) provides sufficient resistance to spray-back and leakage. Such a minimum clearance may be effective for overlapping constant diameters of the elongated shaft 302 and elongated catheter sheath 404.

Referring now to FIG. 9, an embodiment for tip protector sleeve 300B is illustrated. As illustrated in FIG. 9, tip protector sleeve 300B includes an elongated shaft 302, a bead 304B and inner shaft 320. Tip protector sleeve 300B operates similarly as tip protector sleeve 300 with one difference being that backstop 322 is the distal end of inner shaft 320. In such an embodiment, the shape and dimensions of tip protector sleeve 300B are controlled so that bump 408 interferes with movement of the distal end, backstop 322 of inner shaft 320. In such an embodiment, bead 304B may be sized and shaped to act as a stop mechanism that controls the insertion depth of the tip protector sleeve 300B into the working channel and may perform the function as a handle for gripping by the operator

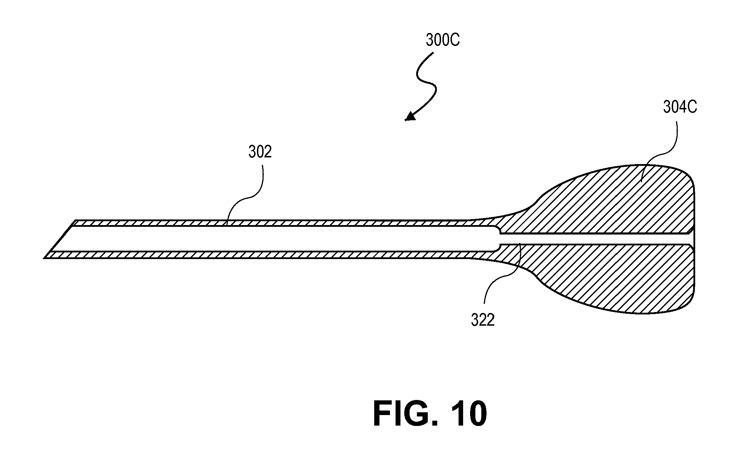

Referring now to FIG. 10, another embodiment for tip protector sleeve 300C is illustrated. As illustrated in FIG. 10, tip protector sleeve 300C includes an elongated shaft 302, a neck portion 332, and a flared portion 304C. Tip protector sleeve 300C operates similarly as tip protector sleeves 300 and 300B. One difference is that the neck portion 332 operates as the backstop for bump 408. The neck portion 332 may be integrally formed with the elongated shaft 302 or be a separate member bonded to the inside diameter of elongated shaft 302. Likewise flared portion 304C may be integrally formed with the elongated shaft 302 or be a separate member bonded to the outside diameter of elongated shaft 302. In such an embodiment, flared portion 304C may be sized and shaped to act as a flanged mechanical stop that controls the insertion depth of the tip protector sleeve 300C into the working channel and may perform the function as a handle for gripping by the operator.
Embodiments of the invention are also envisioned in which the elongated catheter sheath 404 does not have a constant OD along its length. In accordance with some embodiments, the tip protector sleeve 300 may include a change in ID or a valve to accommodate variations in the OD of the elongated catheter sheath 404, or to more effectively seal an elongated catheter sheath 404 with a constant OD. Referring again to FIG. 9, inner shaft 320 is illustrated as having a smaller ID than the ID of elongated shaft 302. In addition to functioning as an interference part, the ID of inner shaft 320 may more effectively accommodate a reduction in OD of the elongated catheter sheath 404. Referring again to FIG. 10, the ID of neck portion 332 may also more effectively accommodate a reduction in OD of the elongated catheter sheath 404 in addition to functioning as an interference part. Thus, a minimum clearance between the ID of inner shaft 320 or ID of neck portion 332 and the OD of the elongated catheter shaft 404 may provide enhanced resistance to fluid spray-back.

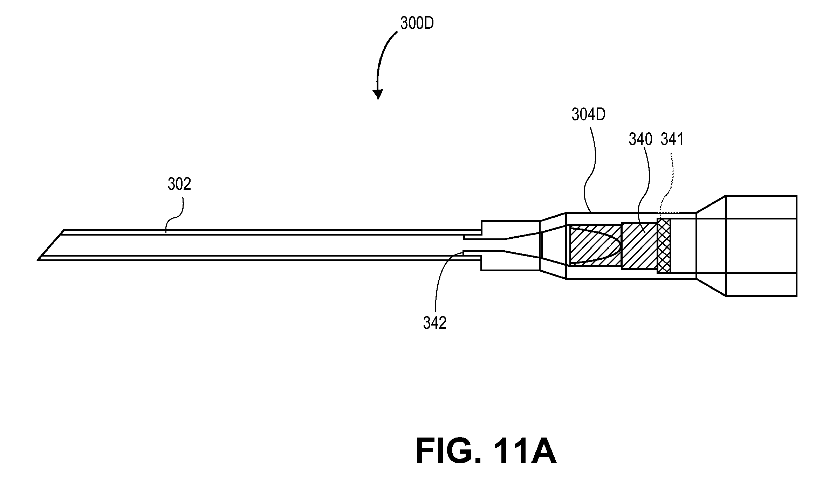

FIGS. 11A-11C are illustrations embodiments of a tip protector sleeve incorporating various sealing valves to reduce the amount of fluid spray-back and leakage between the tip protector sleeve and elongated sheath of the delivery catheter assembly. While illustrated separately, it is understood that the embodiments illustrated in FIGS. 11A-11C may be combined with other embodiments of the invention. More specifically, any of the sealing valves described in reference to the illustrations in FIGS. 11A-11C may be combined with any of the embodiments further describing the proximal-stop and distal-stop positions.

FIG. 11A is an illustration of a tip protector sleeve 300D including an elongated shaft 302, a housing 304D, and valve 340. Housing 304D may function as a stop mechanism that controls the insertion depth of the tip protector sleeve 300D into the working channel and may perform the function as a handle for gripping by the operator. The elongated shaft 302 may be coupled to a distal tip 342 of the housing 304D which may function as a backstop for interference with a bump 408 fixed to the elongated catheter sheath 404 at the distal-stop position. Housing 304D additionally houses valve 340 which is capable of accommodating multiple variations in OD of the elongated catheter sheath 404. Valve 340 may be a silicone valve containing a slit at the distal end that allows for a catheter shaft to pass through it. For example, valve 340 may be a duckbill valve. The silicone material may allow for the slit to conform to different shapes or diameters while providing a seal. Due to the geometry on the distal end of the silicone valve, as fluid tries to pass from distal to proximal, the end of the valve may be pushed closed due to a chamfer on the end of the valve. In an alternative configuration, valve 340 may be a conical valve or slit seal, for example. In some embodiments, tip protector sleeve 300 may additionally include a seal 341 such as an O-ring seal to receive and form a seal around the elongated catheter sheath 404.

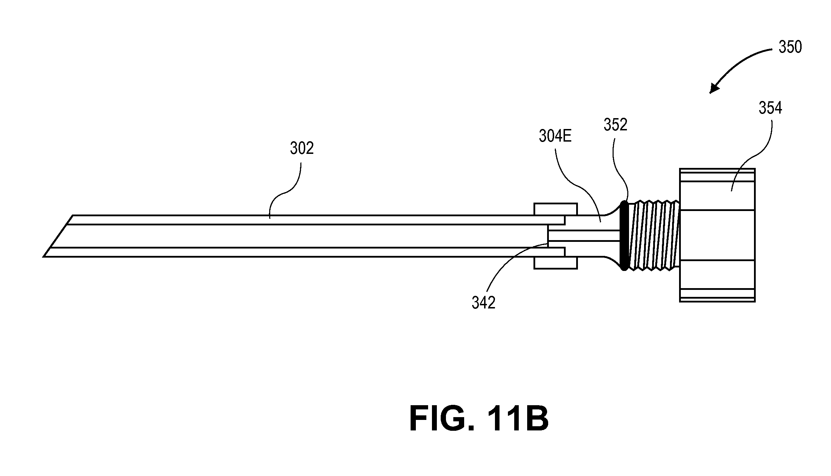

FIG. 11B is an illustration of a tip protector sleeve 300E including an elongated shaft 302, a housing 304E, and a compression valve 350 including an screw cap 354 which may be threaded down onto an O-ring 352 to compress it against the elongated catheter sheath 404. Housing 304E may function as a stop mechanism that controls the insertion depth of the tip protector sleeve 300E into the working channel and may perform the function as a handle for gripping by the operator. The elongated shaft 302 may be coupled to a distal tip 342 of the housing 304E which may function as a backstop for interference with a bump fixed to the elongated catheter sheath at the distal-stop position.

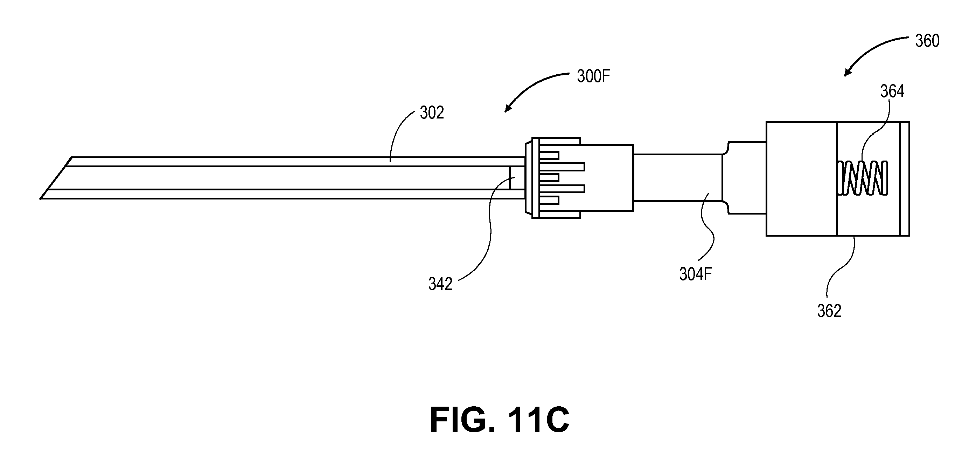

FIG. 11C is an illustration of a tip protector sleeve 300F including an elongated shaft 302, a housing 304F, and a compression valve 360 including a cap 362, a compression spring 364 and a thin walled tube (not illustrated) inside the housing. For example, the thin walled tube may be made of a material such as silicone. Threads between the cap 362 and the housing 304F apply a twist motion to the tube. When the tube is twisted, an inner diameter of the tube tightens like an iris. The compression spring 364 keeps the cap 362 extended and the threads hold the twist or keep the iris closed. When the cap 362 is pushed towards the housing 304F (spring compressed) the cap 362 untwists and the iris opens. In this manner the opening of the iris can be adjusted based upon the OD of the elongated catheter sheath 404. Similar to tip protector sleeves 300D and 300E, housing 304F may function as a stop mechanism that controls the insertion depth of the tip protector sleeve 300F into the working channel and may perform the function as a handle for gripping by the operator. The elongated shaft 302 may be coupled to a distal tip 342 of the housing 304F which may function as a backstop for interference with a bump fixed to the elongated catheter sheath at the distal-stop position.

A delivery catheter assembly in accordance with embodiments of the invention may be utilized to deliver an insert to an ovarian pathway (e.g. a fallopian tube) of a female body. The delivery catheter assembly may protect the tip of an elongated catheter sheath, guidewire, or insert during piercing of a sealing cap and insertion into the working channel and past a valve clamp of a hysteroscope system and reduce the amount of fluid spray-back and leakage associated with inserting a delivery catheter assembly into the working channel of a hysteroscope system. In an embodiment, the delivery catheter assembly includes a control device, an elongated catheter sheath having a distal end and a proximal end connected to the control device, and a tip protector sleeve locked onto the elongated catheter sheath and slideable over a length of the elongated catheter sheath between a proximal-stop position and a distal-stop position along the elongated catheter sheath. The delivery catheter assembly may further include an insert releasably disposed within the elongated catheter sheath. In an embodiment, the insert extends distally beyond the elongated catheter sheath. In an embodiment, the insert includes a preformed bend, as illustrated in FIG. 5, which may be utilized to assist with navigation through a curved portion of a fallopian tube. Upon providing the delivery catheter assembly the operator may grasp a flanged mechanical stop 304 or other suitable portion of the tip protector sleeve 300 to position the tip protector sleeve at the distal-stop position illustrated in FIG. 6. If a sealing valve is present on the tip protection sleeve 300, the sealing valve may then be tightened onto the elongated catheter sheath 404 if necessary to provide an optimal seal to protect against fluid spray-back and leakage.

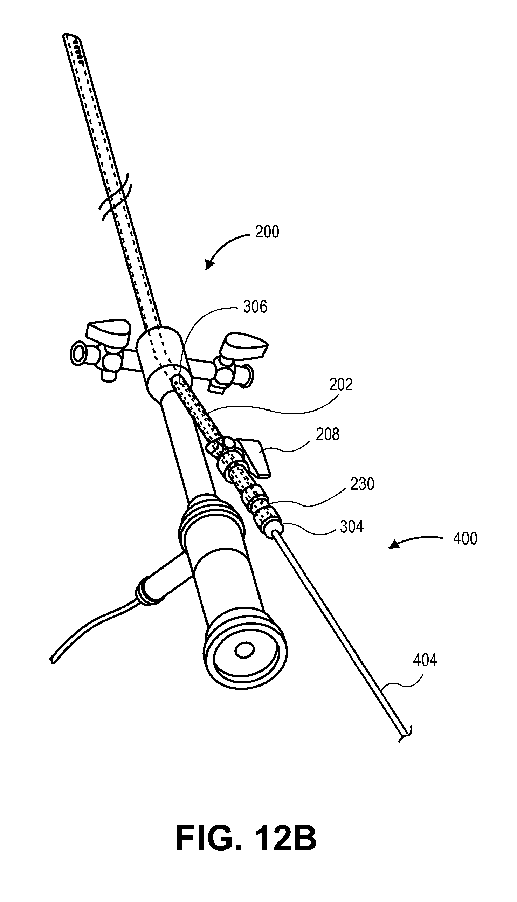

Referring now to FIGS. 12A-12C, the operator may then pierce a proximal seal 232 of a sealing cap 230 with the tip protector sleeve 300 and insert the tip protector sleeve 300 through an access port 206 of a hysteroscope system 200 and into the working channel 202 of the hysteroscope system. During insertion the tip protector sleeve 300 may be advanced past a valve clamp 208 of the hysteroscope system. The tip protector sleeve protects against the possibility of the exposed portion of the insert 406 from catching on the valve clamp 208 and compromising the insert integrity. In accordance with embodiments of the invention, the distal end 410 of the elongated catheter sheath 404 and insert 406 are inserted through the sealing cap 230 and access port 206, and into the working channel 202 of the hysteroscope system simultaneously with the tip protector sleeve 300 in the distal-stop position. The simultaneous insertion of the tip protector sleeve 300 and elongated catheter sheath 404 may avoid a problem of fluid spray-back associated with sequentially inserting an introducer followed by an elongated catheter sheath. In an embodiment, the tip protector sleeve 300 may be advanced into the working channel simultaneously with the elongated catheter sheath and insert 406 until the flanged mechanical stop 304 abuts against the access port 206 or sealing cap 230, if present, as illustrated in FIG. 12B.

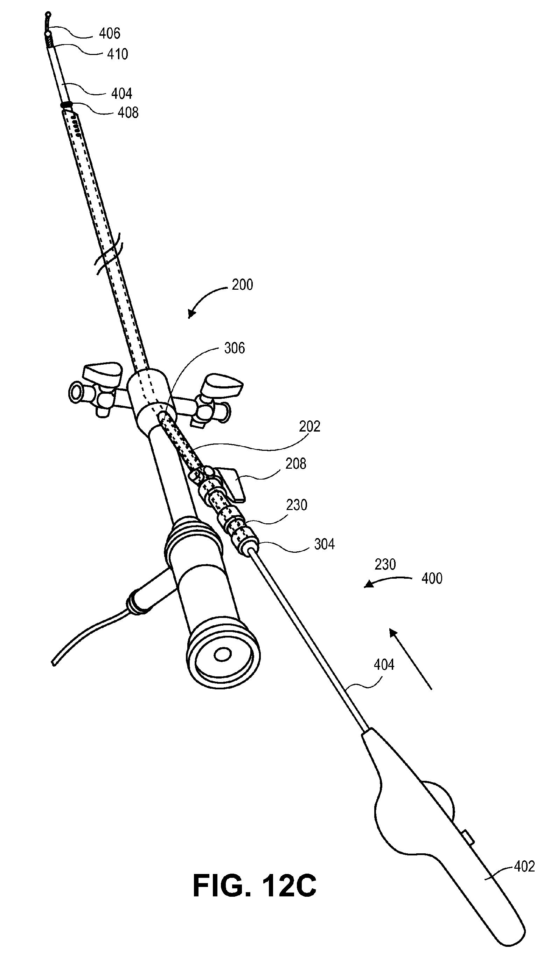

The distal end 410 of the elongated catheter sheath 404 may then be advanced past the hysteroscope system 200 as illustrated in FIG. 12C, and onto a target location with the body lumen. The insert 406 may then be deployed into the body lumen. Depending upon operator preference, the tip protector sleeve 300 may remain inserted in the working channel 202 during the elongated catheter sheath 404 advancement and insert 406 deployment procedures or removed from the working channel 202. In accordance with many embodiments of the invention it is understood that the tip protector sleeve 300 is permanently locked onto the elongated catheter sheath. It is also contemplated that the tip protector sleeve 300 could be removed from the catheter assembly after initially advancing the catheter assembly into the working channel, for example, by including a tear joint in the tip protector sleeve in which the tip protector sleeve can be manually torn off of the elongated catheter sheath by the operator.

In an embodiment, the insert 406 and distal end 410 of the elongated catheter sheath 404 are advanced to the target location within the body lumen while the flanged mechanical stop 304 on the tip protector sleeve 300 abuts the access port 206 or sealing cap 230, if present. The amount of elongated catheter sheath 404 spanning between the flanged mechanical stop 304 and control device 402 may depend upon the procedure and patient's anatomy. It is envisioned that circumstances arise where the operator may wish to insert the entire available working length of the elongated catheter sheath 404 into the patient and advance the control device 402 all the way to the access port or sealing cap, if present. In accordance with embodiments of the invention illustrated in FIGS. 5B-5D, this can be possible by including a cavity 420 in the control device 402 to accommodate the flanged mechanical stop 304.

Once the insert 406 is deployed into the body lumen the delivery catheter assembly may be withdrawn from the working channel of the hysteroscope system. In one embodiment, during withdrawal of the delivery catheter assembly 400 from the working channel, the bump 408 on the elongated catheter sheath 404 may be withdrawn proximally against the backstop of the tip protector sleeve 300 and cause the tip protector sleeve 300 to be withdrawn from the working channel 202 of the hysteroscope system 200. In another embodiment, the flanged mechanical stop 304 on the tip protector sleeve 300 can be fastened to the control device 402. In this manner, during withdrawal of the delivery catheter assembly 400 from the working channel, the tip protector sleeve 300 remains fastened to the control device 402.

In another embodiment, the sealing cap which is fitted over the nozzle 204 containing the access port 206 to the working channel 202 of the hysteroscope system may be configured to further prevent against spray-back and leakage of distention fluid, and additionally may be configured to be compatible with a plurality of commercially available hysteroscope systems having nozzles of different outside dimensions (e.g. width or diameter).

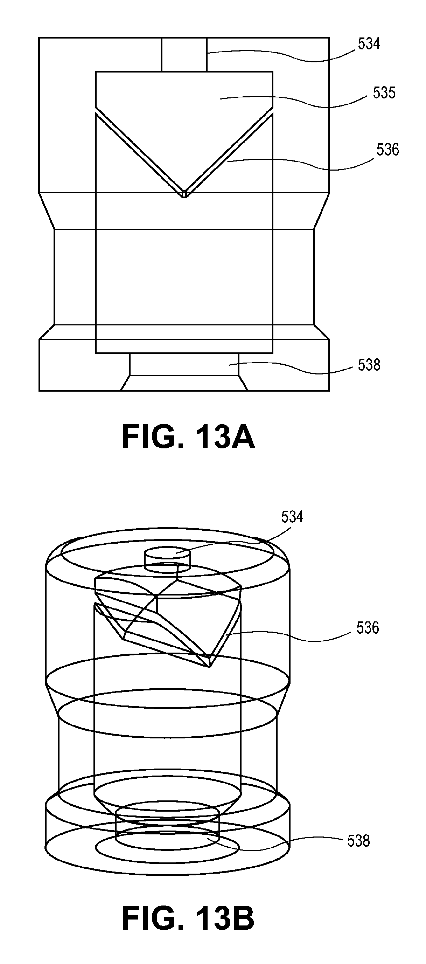

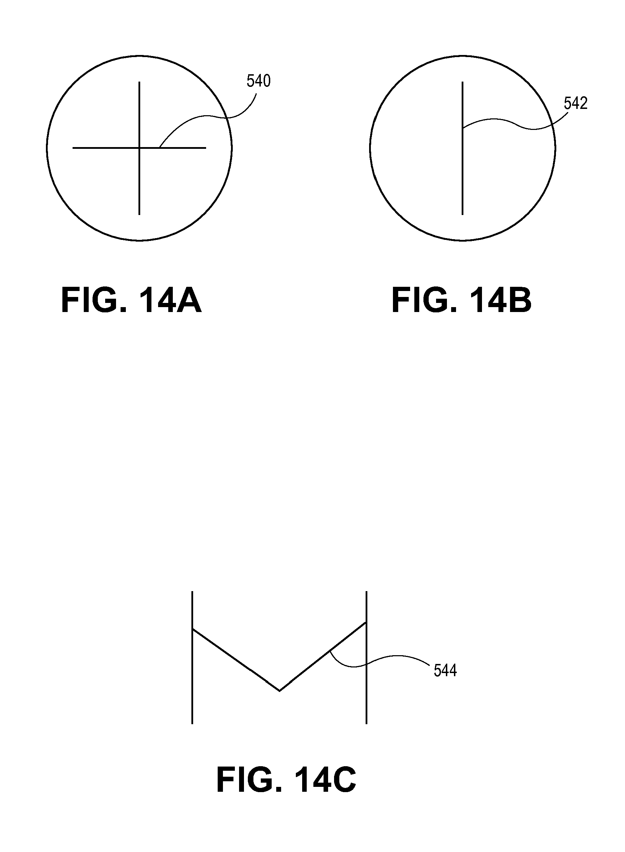

Referring now to FIGS. 13A-13B, a sealing cap 530 is illustrated which includes a distal seal 538 and a double seal formed of a proximal seal 534 and a self closing seal 536 in series so that one of the seals 534, 536 is always providing a seal to protect against leakage and spray-back. Proximal seal 534 and self closing seal 536 may be separated by a chamber 535. The proximal seal 534 may be and open seal, such as an O-ring, and be configured to seal against an object such as an elongated sheath 104 of a delivery catheter assembly such as that illustrated in FIG. 1, an introducer, such as the DryFlow (TM) introducer available from Conceptus, Inc. of Mountain View, California, or the elongated shaft 302 of a tip protector sleeve 300 on a delivery catheter assembly as illustrated in FIG. 12A-12C. The distal seal 538 may be an open seal, such as an O-ring, and be configured to be fitted over and seal against a nozzle 204 containing an access port 206 for a variety of commercially available hysteroscope systems 200. Self closing seal 536 is configured so that it may be pierced yet also be self closing and thereby prevent fluid leakage and spray-back when an object is not inserted therethrough. In certain embodiments, the self closing seal 536 may self seal due to distention fluid pressure in the working channel of the hysteroscope system. In the specific embodiment illustrated in FIGS. 13-13B, self closing seal 536 is illustrated as a duckbill valve, though other types of self closing seals may be used. For example, referring to to FIG. 14A-14C, the self closing seal 536 can be in the form of a cross-hatch seal 540, slit seal 542, or a duckbill valve or conical valve 544, for example.

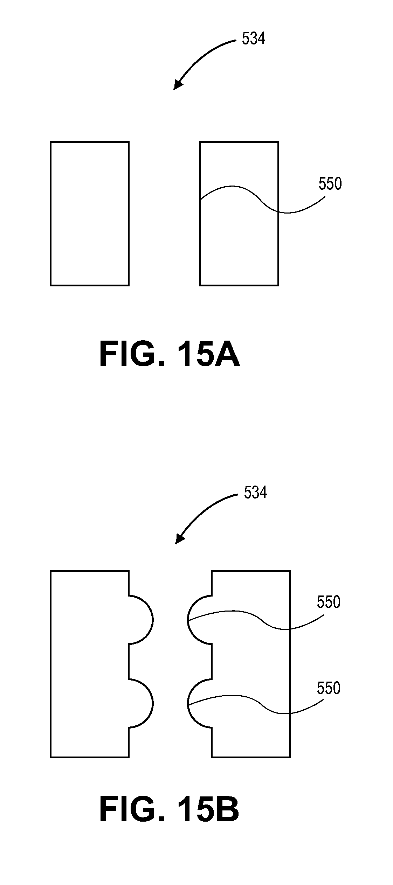

Referring now to FIGS. 15A-15B close-up views of proximal seal 534 are illustrated in accordance with embodiments of the invention. As illustrated in FIG. 15A, proximal seal 534 may include an O-ring 550 with an inside dimension (e.g. width or diameter) configured to make contact with and seal against the object inserted therethrough. While O-ring 550 is illustrated as having a uniform inside dimension, it is to be appreciated that the inside dimension of O-ring is not necessarily constant ant may be rounded, and the amount of contact surface area of the O-ring against the object inserted therethrough can be modulated by adjusting the shape or height of the O-ring. For example, FIG. 15B illustrates a proximal seal 534 including a plurality of rounded O-rings 550 configured to make contact with and seal against the object inserted therethrough. In such an embodiment, the plurality of O-rings may reduce the overall surface area the proximal seal 534 makes contact with the object which may reduce friction and allow for more controlled manipulation of the object (e.g. introducer or delivery catheter assembly) by the operator. While two O-rings are illustrated in FIG. 15B, it is understood that one or more O-rings may be included.

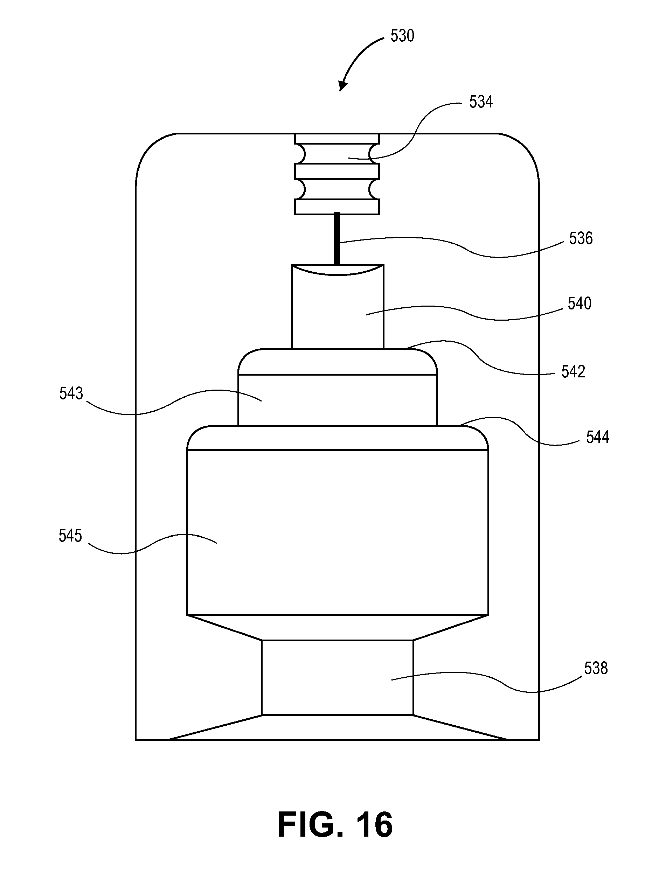

In accordance with embodiments of the invention, the sealing cap 530 may be configured to be compatible with a plurality of different commercially available hysteroscope systems having nozzles of different outside dimensions. As illustrated in FIG. 16, sealing cap 530 may include a proximal seal 534, self closing seal 536 and distal seal 538 as previously described. In the particular embodiment illustrated in FIG. 16, proximal seal 534 includes a plurality of O-rings as described with regard to FIG. 15B, and self closing seal 536 is a slit seal as described with regard to FIG. 14B, though it is to be appreciated that embodiments of the invention are not limited to the specific seals illustrated, and that other combinations of seals are contemplated in accordance with embodiments of the invention. Still referring to FIG. 16, sealing cap 530 may include a plurality of stepped shoulders 542, 544 of different dimensions (e.g. width or diameter) to accommodate the outside dimensions (e.g. width or diameter) of different nozzles of different commercially available hysteroscope systems. While two shoulders 542, 544 are illustrated, it is understood that one or more shoulders may be present. In application, the sealing cap 530 is fitted over the nozzle 204 of a hysteroscope system 200 and advanced over the nozzle 204 until the access port 206 abuts against one of the shoulders 542, 544. In accordance with some embodiments, the inside dimension of each chamber region 543, 545 corresponding to each shoulder 542, 544 is only slightly larger than the outside dimension of the nozzle 204 region including the access port 206 for a commercially available hysteroscope system 200. In this manner, the inside dimension of chamber regions 543, 545 may assist with alignment of the sealing cap with the working channel of a hysteroscope system and better enable insertion of an object (e.g. introducer or delivery catheter assembly) into the working channel without damaging the distal tip of object, such as, but not limited to insert 106, 406 of a delivery catheter assembly.

Sealing cap 530 may additionally be provided with a separation chamber 540 distally adjacent to the self closing seal 536 and spatially separating the self closing seal 536 from the plurality of stepped shoulders 542, 544. This spatial separation prevents the nozzle 204 from abutting against the self closing seal 536, thereby reducing the risk of the access port 206 making it more difficult for the delivery catheter assembly to pierce the self closing seal 536. In this manner, the spatial separation may better enable insertion of object through the self closing seal 536 without damaging the distal tip of object, such as, but not limited to insert 106, 406 of a delivery catheter assembly.

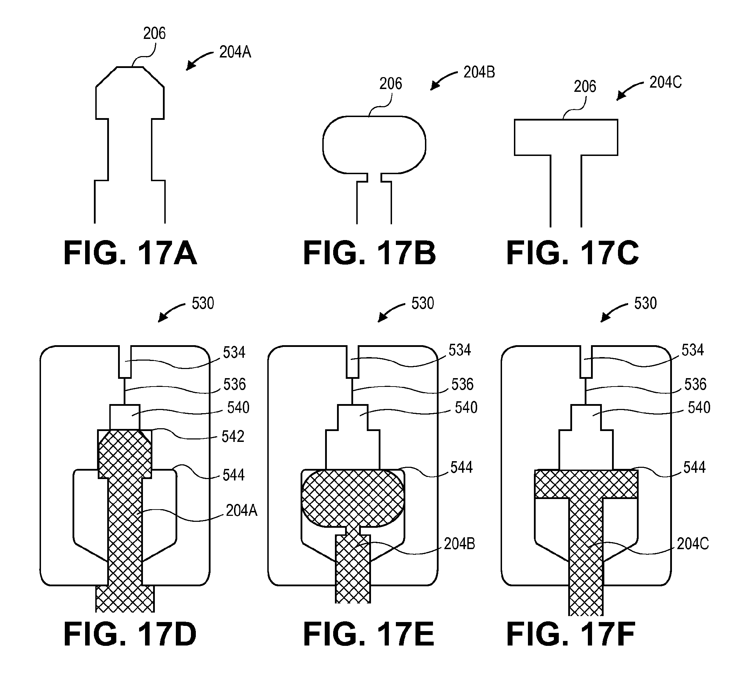

Illustrations of nozzles 204A-204C of various outside dimensions are illustrated in FIGS. 17A-17C prior to being fitted with a sealing cap 530, and illustrated in FIGS. 17D-17F, respectively, after fitting a sealing cap 530 over the respective nozzle 204A-204C. As illustrated, nozzle 204A having a smaller outside dimension may abut against shoulder 542 of sealing cap 530. In certain embodiments, the outside dimension of nozzle 204A may be only slightly smaller than the inside dimension of chamber region 543 associated with shoulder 542. Nozzles 204B, 204C likewise are illustrated as having a larger outside dimension abutting against shoulder 544 of sealing cap 530. In certain embodiments, a portion of the outside dimension of nozzles 204B, 204C may be only slightly smaller than the insider dimension of chamber region 545 associated with shoulder 544. In all illustrations, the nozzles 204A-204C are spatially separated from self closing seal 536 by separation chamber 540 distally adjacent to the self closing seal 536.

Sealing cap 530 may be formed of any suitable material known in the art possessing sufficient flexibility, strength and tear resistance so that the sealing cap 530 may be fixed over and form a seal around a variety of different types of nozzles having different outside dimensions, and also be pierced by and form seals around an introducer or delivery catheter assembly. Suitable materials may include silicones, rubber and other plastics. In certain embodiments, the distal region of a nozzle 204 has an outside dimension which is greater than the inside dimension of distal seal 538 of the sealing cap 530. In such embodiments, the sealing cap is formed of a material which is flexible enough to allow the insertion of the nozzle 204 region having the larger outside dimension through the distal seal 538 having the smaller outside dimension, and to form a tight seal around the nozzle 204 which prevents against leakage and spray-back. In such embodiments, inside shoulders 542, 544 and the corresponding chambers 543, 545 may have inside dimensions which are larger than the inside dimension of seal 538 in order to accommodate and align the nozzle 204. Proximal seal 534 may also have a smaller inside dimension than distal seal 538 and inside shoulders 542, 544, and the corresponding chambers 543, 545. In one variation, sealing cap 530 may be formed of or include a coating of a hydrophilic material in order to soak up small amounts of distention fluid thereby providing additional protection against leakage.

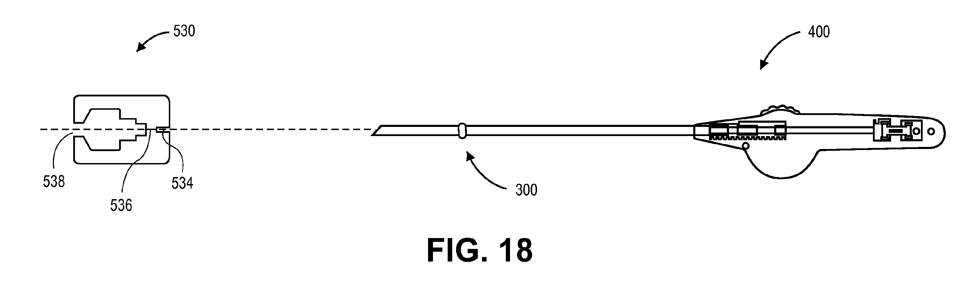

In accordance with embodiments of the invention, multiple components which may reduce or eliminate fluid leakage or spray-back are provided together in a kit. In one embodiment illustrated in FIG. 18, a kit may include a delivery catheter assembly 400 including a tip protector sleeve 300 as previously described and a sealing cap 530 as described with regard to FIGS. 13A-17F. In such a kit, distal seal 538 is configured to be fitted over a nozzle of a hysteroscope system, self closing seal 536 is configured to protect against leakage and spray-back when a delivery catheter assembly 400 is not inserted, and proximal seal 534 is configured to protect against leakage and spray-back when the delivery catheter assembly is inserted therethrough. As previously described, sealing cap 530 may include a plurality of shoulders to fit a variety of nozzles of different outside dimensions. In operation, the distal seal 538 of a sealing cap 530 is fitted over a nozzle housing a working channel of a hysteroscope system. The tip protector sleeve 300 of a delivery catheter assembly 400 is then inserted through the proximal seal 536 and self closing seal 536 and into the working channel 202 of the hysteroscope system.

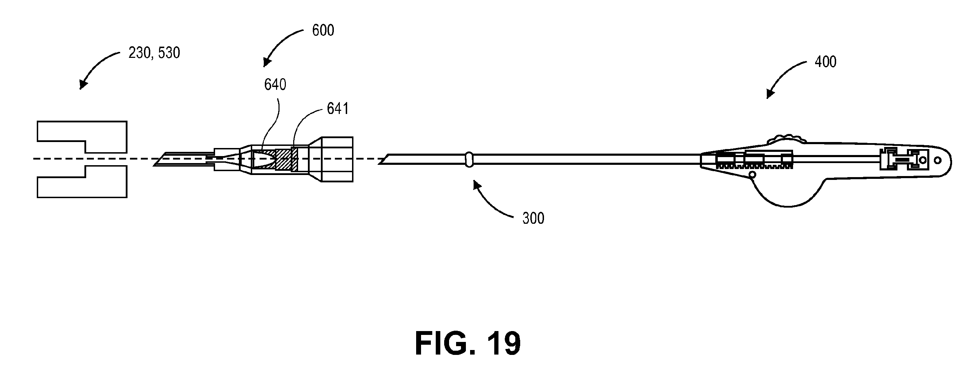

In one embodiment illustrated in FIG. 19 a kit may include a delivery catheter assembly including a tip protector sleeve 300 as previously described, an intruder 600 such as a DryFlow (TM) introducer, and a sealing cap. In such an embodiment, introducer 600 may resemble the tip protector sleeve 300D illustrated in FIG. 11A. For example, introducer 600 may include a valve 640 such a slit seal, duckbill valve, or conical valve. Such a valve may be formed of a silicone material, for example, which conforms to different shapes or diameters while providing a seal. Due to the geometry, valve 640 is self closing. As fluid tries to pass from distal to proximal, the end of the valve 640 may be pushed closed due to a chamfer on the end of the valve to protect against leakage and spray-back. Introducer 600 may additionally include a seal 641 such as an O-ring to receive and form a seal around tip protector sleeve 300 to provide additional protection against leakage and spray-back. In this manner, valve 640 protects against leakage and spray-back when tip protector sleeve 300 is not yet inserted, and seal 641, in addition to valve 640, protects against leakage and spray-back when tip protector sleeve 300 is inserted. Sealing cap may be a conventional sealing cap 230 including a distal end which can be fitted over a nozzle of a hysteroscope system, and a proximal end which may be pierced by the delivery catheter assembly. In another embodiment, sealing cap may be sealing cap 530 as described with regard to FIGS. 13A-17F in order to provide additional protection against fluid leakage and spray-back. In operation, the distal end of a sealing cap 230. 530 is fitted over a nozzle housing a working channel of a hysteroscope system. The introducer 600 is then inserted through the proximal end of the sealing cap 230, 530 and into the working channel 202 of the hysteroscope system. The tip protector sleeve 300 of a delivery catheter assembly 400 is then inserted through the introducer 600.

In the foregoing specification, various embodiments of the invention have been described. It will, however, be evident that various modifications and changes may be made thereto without departing from the scope of the invention as set forth in the appended claims. The specification and drawings are, accordingly, to be regarded in an illustrative sense rather than a restrictive sense. Hence, the scope of the present invention is limited solely by the following claims.

## Claims

1. A sealing cap (530) comprising:
a proximal seal (534); and
a distal seal (538) configured to be fitted over a nozzle housing a working channel of a hysteroscope system;
wherein the proximal seal (534) is configured to reduce fluid leakage when a delivery system (400) is inserted through the proximal seal (534),
**characterised in that** the sealing cap (530) further comprises a self closing seal (536) which is configured to reduce fluid leakage when a delivery system is not inserted into the sealing cap.

2. The sealing cap of claim 1, wherein the proximal seal is an open seal.

3. The sealing cap of claim 2, wherein the open seal comprises an O-ring (550).

4. The sealing cap of claim 3, wherein the open seal comprises a plurality of O-rings (550).

5. The sealing cap of claim 1, wherein the self closing seal comprises a slit seal (542).

6. The sealing cap of claim 1, wherein the self closing seal comprises a duckbill valve (544) or a conical valve (544).

7. The sealing cap of claim 1, further comprising:
a plurality of shoulders (542, 544) of different dimensions; and
a separation chamber (540) distally adjacent to the self closing seal (536) which spatially separates the self closing seal from the plurality of shoulders (542, 544).

8. The sealing cap of claim 7, wherein the plurality of shoulders are arranged in a series of steps.

9. The sealing cap of claim 7, wherein the plurality of shoulders have dimensions that are greater than an inside dimension of the distal seal (538).

10. A kit comprising:
the sealing cap of claim 7; and
a delivery catheter assembly (400) comprising:
a control device (402);
an elongated catheter sheath (404) having a distal end and a proximal end connected to the control device; and
a tip protector sleeve (300) locked onto the elongated catheter sheath and slideable over a length of the elongated catheter sheath between a proximal-stop position and a distal-stop position along the elongated catheter sheath.

11. The kit of claim 10, wherein the plurality of shoulders of the cap are arranged in a series of steps.

12. The kit of claim 10, wherein the delivery catheter system further comprises an interference stop (408) which determines the distal-stop position and prevents the tip protector sleeve from sliding off of the distal end of the elongated catheter sheath.

13. The kit of claim 10 further comprising:
an introducer (600) comprising an elongated sheath and a valve (640).

14. The kit of claim 13, wherein the introducer valve (640) is selected from the group consisting of a slit seal, duckbill valve and conical valve.

15. The kit of claim 14, wherein the introducer further comprise an O-ring which forms a seal (641) around the tip protector sleeve when inserted through the introducer.

16. The kit of claim 13, wherein the delivery catheter system further comprises an interference stop (408) which determines the distal-stop position and prevents the tip protector sleeve from sliding off of the distal end of the elongated catheter sheath, and the interference stop (408) comprises a male interference part which interferes with sliding of a female interference part (308, 312) over the elongated catheter sheath.

## Patentansprüche

1. Dichtungskappe (530), umfassend:
eine proximale Dichtung (534); und
eine distale Dichtung (538) zur Anbringung über einer Düse, die einen Arbeitskanal eines Hysteroskopsystems beherbergt;
worin die proximale Dichtung (534) ausgebildet ist, Austreten von Flüssigkeit bei Einführung eines Abgabesystems (400) durch die proximale Dichtung (534) zu reduzieren,
**dadurch gekennzeichnet, dass** die Dichtungskappe (530) ferner eine selbstschließende Dichtung (536) umfasst, die ausgebildet ist, Austreten von Flüssigkeit zu reduzieren, wenn kein Abgabesystem in die Dichtungskappe eingeführt wird.

2. Dichtungskappe nach Anspruch 1, worin die proximale Dichtung eine offene Dichtung ist.

3. Dichtungskappe nach Anspruch 2, worin die offene Dichtung einen O-Ring (550) umfasst.

4. Dichtungskappe nach Anspruch 3, worin die offene Dichtung eine Vielzahl von O-Ringen (550) umfasst.

5. Dichtungskappe nach Anspruch 1, worin die selbstschließende Dichtung eine Spaltdichtung (542) umfasst.

6. Dichtungskappe nach Anspruch 1, worin die selbstschließende Dichtung ein Entenschnabelventil (544) oder ein konisches Ventil (544) umfasst.

7. Dichtungskappe nach Anspruch 1, ferner umfassend:
eine Vielzahl von Schultern (542, 544) mit unterschiedlichen Abmessungen; und
eine Trennkammer (540) distal neben der selbstschließenden Dichtung (536), welche die selbstschließende Dichtung räumlich von der Vielzahl von Schultern (542, 544) trennt.

8. Dichtungskappe nach Anspruch 7, worin die Vielzahl von Schultern in einer Reihe von Stufen angeordnet ist.

9. Dichtungskappe nach Anspruch 7, worin die Vielzahl von Schultern Abmessungen aufweisen, die größer als eine Innenabmessung der distalen Dichtung (538) sind.

10. Kit, umfassend:
die Dichtungskappe nach Anspruch 7; und
eine Abgabekatheteranordnung (400), umfassend:
eine Steuervorrichtung (402);
eine langgestreckte Katheterhülle (404) mit einem distalen Ende und einem proximalen Ende, das mit der Steuervorrichtung verbunden ist; und
eine Spitzenschutzhülse (300), die auf der langgestreckten Katheterhülle verriegelt ist und über eine Länge der langgestreckten Katheterhülle zwischen einer proximalen Anschlagstellung und einer distalen Anschlagstellung entlang der langgestreckten Katheterhülle verschiebbar ist.

11. Kit nach Anspruch 10, worin die Vielzahl von Schultern der Kappe in einer Reihe von Stufen angeordnet ist.

12. Kit nach Anspruch 10, worin das Abgabekathetersystem ferner eine Interferenzsperre (408) umfasst, welche die distale Anschlagsstellung festlegt und verhindert, dass die Spitzenschutzhülse vom distalen Ende der langgestreckten Katheterhülle abgleitet.

13. Kit nach Anspruch 10, ferner umfassend:
ein Einführungsinstrument (600), das eine langgestreckte Hülle und ein Ventil (640) umfasst.

14. Kit nach Anspruch 13, worin das Ventil (640) des Einführungsinstruments aus der aus einem Spaltventil, Entenschnabelventil und konischen Ventil bestehenden Gruppe ausgewählt ist.

15. Kit nach Anspruch 14, worin das Einführungsinstrument ferner einen O-Ring umfasst, der bei der Einführung durch das Einführungsinstrument eine Dichtung (641) um die Spitzenschutzhülse bildet.

16. Kit nach Anspruch 13, worin das Abgabekathetersystem ferner eine Interferenzsperre (408) umfasst, welche die distale Anschlagstellung festlegt und verhindert, dass die Spitzenschutzhülse vom distalen Ende der langgestreckten Katheterhülle abgleitet, und worin die Interferenzsperre (408) einen männlichen Interferenzteil umfasst, der eine Verschiebung eines weiblichen Interferenzteils (308, 312) über die langgestreckte Katheterhülle behindert.

## Revendications

1. Embout d'étanchéité (530), comprenant:
un joint proximal (534); et
un joint distal (538) configuré de manière à être agencé sur une buse renfermant un canal de travail d'un système d'hystéroscopie,
dans lequel le joint proximal (534) est configuré de manière à réduire toute fuite de fluide lorsqu'un système d'administration (400) est inséré à travers le joint proximal (534),
**caractérisé en ce que** l'embout d'étanchéité (530) comprend en outre un joint à fermeture automatique (536) qui est configuré pour réduire toute fuite de fluide lorsqu'un système d'administration (400) n'est pas inséré dans l'embout d'étanchéité.

2. Embout d'étanchéité selon la revendication 1, dans lequel le joint proximal est un joint ouvert.

3. Embout d'étanchéité selon la revendication 2, dans lequel le joint ouvert comprend un joint torique (550).

4. Embout d'étanchéité selon la revendication 3, dans lequel le joint ouvert comprend une pluralité de joints toriques (550).

5. Embout d'étanchéité selon la revendication 1, dans lequel le joint à fermeture automatique comprend un joint fendu (542).

6. Embout d'étanchéité selon la revendication 1, dans lequel le joint à fermeture automatique comprend une soupape en bec de canard (544) ou une soupape conique (544).

7. Embout d'étanchéité selon la revendication 1, comprenant en outre:
une pluralité d'épaulements (542, 544) de dimensions différentes; et
une chambre de séparation (540) adjacente à distance au joint à fermeture automatique (536) qui sépare spatialement le joint à fermeture automatique de la pluralité d'épaulements (542, 544).

8. Embout d'étanchéité selon la revendication 7, dans lequel la pluralité d'épaulements sont agencées en une série de gradins.

9. Embout d'étanchéité selon la revendication 7, dans lequel la pluralité d'épaulements présentent des dimensions qui sont plus grandes qu'une dimension intérieure du joint distal (538).

10. Kit, comprenant:
l'embout d'étanchéité selon la revendication 7; et
un ensemble de cathéter d'administration (400), comprenant:
un dispositif de commande (402);
une gaine de cathéter allongée (404) présentant une extrémité distale et une extrémité proximale connectée au dispositif de commande; et
un manchon de protection de pointe (300) verrouillé sur la gaine de cathéter allongée et pouvant coulisser sur une longueur de la gaine de cathéter allongée entre une position d'arrêt proximale et une position d'arrêt distale le long de la gaine de cathéter allongée.

11. Kit selon la revendication 10, dans lequel la pluralité d'épaulements de l'embout sont agencés en une série de gradins.

12. Kit selon la revendication 10, dans lequel le système de cathéter d'administration comprend en outre un arrêt d'interférence (408) qui détermine la position d'arrêt distale et qui empêche le manchon de protection de pointe de glisser de l'extrémité distale de la gaine de cathéter allongée.

13. Kit selon la revendication 10, comprenant en outre un introducteur (600) comprenant une gaine allongée et une soupape (640).

14. Kit selon la revendication 13, dans lequel la soupape d'introducteur (640) est sélectionnée dans le groupe comprenant un joint fendu, une soupape en bec de canard et une soupape conique.

15. Kit selon la revendication 14, dans lequel l'introducteur comprend en outre un joint torique qui forme un joint (641) autour du manchon de protection de pointe lorsqu'il est inséré à travers l'introducteur.

16. Kit selon la revendication 13, dans lequel le système de cathéter d'administration comprend en outre un arrêt d'interférence (408) qui détermine la position d'arrêt distale et qui empêche le manchon de protection de pointe de glisser de l'extrémité distale de la gaine de cathéter allongée, et l'arrêt d'interférence (408) comprend une partie d'interférence mâle qui interfère avec le coulissement d'une partie d'interférence femelle (308, 312) sur la gaine de cathéter allongée.
